# EUROPÄISCHE PATENTSCHRIFT

(11) **EP 4 025 157 B1**
(45) Veröffentlichungstag und Bekanntmachung des Hinweises auf die Patenterteilung: **11.06.2025**
(21) Anmeldenummer: 20785898.6
(22) Anmeldetag: 02.09.2020
(51) Int. Cl.: A61C 13/00, A61C 8/00

(54) **VERFAHREN ZUR HERSTELLUNG EINES COMPUTERMODELLS FÜR EIN ABUTMENT UND EINES ABUTMENTS**
METHOD FOR PRODUCING A COMPUTER MODEL FOR AN ABUTMENT AND FOR PRODUCING AN ABUTMENT
PROCÉDÉ DE FABRICATION D'UN MODÈLE INFORMATIQUE DE BUTÉE, ET DE FABRICATION D'UNE BUTÉE

(30) Priorität: 03.09.2019 AT 507672019
(43) Veröffentlichungstag der Anmeldung: 13.07.2022
(73) Patentinhaber: Eap® Produktions- Und Patentverwertungs- GmbH, 6060 Hall in Tirol (AT)
(72) Erfinder: KERN, Mario, 6233 Kramsach (AT)
(74) Vertreter: Torggler & Hofmann Patentanwälte - Innsbruck
(86) Internationale Anmeldenummer: PCT/AT2020/060328
(87) Internationale Veröffentlichungsnummer: WO 2021/042149

(56) Entgegenhaltungen:
- EP-B1- 2 204 138
- US-A1- 2012 072 178
- US-A1- 2013 189 646

## Beschreibung

Die Erfindung betrifft ein Verfahren mit den Merkmalen des Oberbegriffs des Anspruchs 1 und/oder des Anspruchs 2 und ein Verfahren zur Herstellung eines Abutments sowie ein Computerprogrammprodukt, ein computerlesbares Speichermedium, einen computerlesbaren Datenträger und ein Datenträgersignal, das ein solches Computerprogrammprodukt überträgt und einen Computer, welcher zur Durchführung eines der Verfahren und/oder zur Ausführung eines solchen Computerprogrammprodukts konfiguriert ist.

Unter einem Abutment (auf Deutsch manchmal auch als "Aufsatz" bezeichnet) wird in der Zahnmedizin das Verbindungselement zwischen einem stiftförmigen Zahnimplantat und einer prothetischen Versorgung (z. B. Einzelzahnversorgung, Brückenversorgung, Prothese usw.) verstanden. Die Verbindung mit der prothetischen Versorgung kann unmittelbar oder über eine Mesostruktur erfolgen.

Ein Abutment geht aus EP 2 825 124 B1 hervor. Das in diesem Dokument gezeigte Abutment weist eine Plattform mit einer tellerartigen Ausbildung der koronal schauenden Plattformoberfläche auf, welche das Anwachsen von Zellen begünstigt und eine Abstützung einer Mesostruktur oder einer prothetischen Versorgung auf der koronal schauenden Plattformoberfläche gestattet. Durch die Möglichkeit einer nachträglichen Bearbeitung des Abutments ergibt sich eine Individualisierbarkeit des Abutments für einen bestimmten Patienten.

US 2013/189646 A1, US 2012/072178 A1 und EP 2 204 138 B1 werden als relevanter Stand der Technik zitiert.

Unter einem Emergenzprofil versteht man in der zahntechnischen Implantatversorgung den dreidimensionalen Verlauf des Zahnfleisches dort, wo das Abutment zu platzieren ist, in einem koronal verlaufenden Bereich ausgehend vom Zahnimplantat bis zur Zahnfleischgrenze.

Neben vorgefertigten Standard-Abutments, welche im Dentallabor aufwändig manuell nachbearbeitet werden müssen, um sie an die konkreten Gegebenheiten an der Versorgungsstelle im Kiefer des Patienten anzupassen, ist es bereits bekannt, unmittelbar patientenindividualisierte Abutments herzustellen. Dies erfolgt über ein durch einen Computer lesbares dreidimensionales Computermodell des herzustellenden patientenindividualisierten Abutments. Das dreidimensionale Computermodell wird entweder auf Basis eines analogen Kieferabdrucks des Kiefers des Patienten, welcher digitalisiert wird, hergestellt oder unmittelbar in digitaler Form (z. B. unter Verwendung eines intraoralen Scanners oder Laborscanners) bereitgestellt.

Durch einen Bediener (z. B. Zahntechniker oder Zahnarzt) oder durch einen Algorithmus wird dort, wo das Abutment zu platzieren ist, der für den Patienten individuelle Zahnfleischverlauf definiert.

Durch den Bediener oder einen Algorithmus ist außerdem die dreidimensionale Geometrie des Abutments oberhalb einer durch den individuellen Zahnfleischverlauf vorgegebenen Präparationsgrenze zu definieren. Die Definition der dreidimensionalen Geometrie des Abutments kann umfassen:
- Definition einer Lage eines im Abutment verlaufenden Schraubenkanals zur Befestigung des Abutments an ein stiftförmiges Zahnimplantat. Üblicherweise ist die Lage relativ zu einer Ausrichtung des stiftförmigen Zahnimplantats definiert.
- Definition einer Breite einer koronal schauenden Plattformoberfläche des Abutments.
- Definition einer Höhe des Abutments.
- Definition einer Breite einer den Schraubenkanal aufweisenden zentralen Erhebung des Abutments

Außerdem ist das Emergenzprofil des Abutments durch einen Bediener des Computers oder durch einen Algorithmus zu definieren.

Das Emergenzprofil, d. h. die Außengeometrie des Abutments unterhalb der Präparationsgrenze bis zum stiftförmigen Zahnimplantat, wurde bisher mit einer Standardform vorgegeben und der Bereich zwischen dem Emergenzprofil und dem Schraubenkanal wurde als ausgefüllter Körper definiert, sodass der Bediener des Computers hier keinerlei Handlungsspielraum hatte. Als Resultat wurde in der Praxis häufig eine an sich zu tief verlaufende Präparationsgrenze gewählt, um jenen Bereich, der durch den Bediener oder den Algorithmus gewählt oder definiert werden konnte, zu vergrößern. Dies bringt das Problem mit sich, dass sich die sogenannte Klebefuge zwischen dem Abutment und der prothetischen Versorgung in Richtung des Zahnimplantats und damit des Kieferknochens des Patienten verschiebt, was destruktive Folgen für das Zahnfleisch und den Kieferknochen hat.

Aufgabe der Erfindung ist die Bereitstellung eines Verfahrens zur Herstellung eines dreidimensionalen Computermodells eines für einen Patienten bestimmten patientenindividuellen Abutments für ein Zahnimplantat mittels eines Computers und eines Verfahrens zur Herstellung eines solchen Abutments für ein Zahnimplantat, bei welchem das Computermodell bzw. das nach diesem hergestellte Abutment besser verträglich für das Zahnfleisch und den Kieferknochen des Patienten ist.

Diese Aufgabe wird durch ein Verfahren mit den Merkmalen des Anspruchs 1 und/oder des Anspruchs 2 und durch ein Verfahren mit den Merkmalen des Anspruchs 7 gelöst.

Weiters soll die Erfindung ein Computerprogrammprodukt umfassend Befehle zur Ausführung des Verfahrens, ein computerlesbares Speichermedium und einen computerlesbaren Datenträger umfassend solche Befehle sowie ein Datenträgersignal, das ein solches Computerprogrammprodukt überträgt, und einen Computer zur Durchführung eines der erfindungsgemäßen Verfahren und/oder zur Ausführung des erfindungsgemäßen Computerprogrammprodukts bereitstellen.

Diese Aufgabe wird durch ein Computerprogrammprodukt mit den Merkmalen des Anspruchs 8, ein computerlesbares Speichermedium mit den Merkmalen des Anspruchs 9, einen computerlesbaren Datenträger mit den Merkmalen des Anspruchs 10, ein Datenträgersignal mit den Merkmalen des Anspruchs 11 und einen Computer mit den Merkmalen des Anspruchs 12 gelöst.

Die Schritte der erfindungsgemäßen Verfahren werden außerhalb eines Körpers des Patienten durchgeführt, da die Erfindung von einer bereits vorliegenden dreidimensionalen Darstellung eines Kiefers des Patienten zumindest in jenem Bereich des Kiefers, wo das Abutment zu platzieren ist, ausgeht.

Vorteilhafte Ausführungsformen der Erfindung sind in den abhängigen Ansprüchen definiert.

Gemäß einer ersten Variante der Erfindung ist unterhalb der Präparationsgrenze für die dreidimensionale Geometrie der Plattform eine tellerartige Ausbildung der koronal schauenden Plattformoberfläche vorgesehen, wie sie an sich aus EP 2 825 124 B1 bekannt ist. Die tellerartige Ausbildung weist eine vorteilhaft weit weg vom Zahnimplantat angeordnete Klebefuge zwischen dem Abutment und der prothetischen Versorgung auf. Weil weiters vorgesehen ist, dass durch einen Bediener des Computers ausgehend vom definierten Emergenzprofil, d. h. unterhalb der Präparationsgrenze, eine Materialstärke der Plattform des Abutments gewählt wird, entfällt für den Bediener des Computers die Veranlassung, zur Vergrößerung seines Gestaltungsspielraums eine an sich zu tief verlaufende Präparationsgrenze zu wählen, da die Erfindung dem Bediener von Vornherein eine Gestaltung der dreidimensionalen Geometrie des Abutments unterhalb der Präparationsgrenze gestattet. Um hier unvorteilhafte Gestaltungen zu vermeiden, die bei einem nach dem durch den Computer bereitgestellten dreidimensionalen Computermodell hergestellten Abutment zu einem Versagen des Abutments führen könnten, sieht die Erfindung vor, dass die dreidimensionale Geometrie des Abutments unterhalb der Präparationsgrenze durch den Bediener nur innerhalb eines in einem elektronischen Speicher hinterlegten, vorgegebenen Bereichs gewählt werden kann.

An sich wäre eine tellerartige Ausbildung der koronal schauenden Plattformoberfläche allein ausreichend. Bevorzugt ist jedoch auch eine in koronaler Richtung gewölbt ausgebildete Unterseite der Plattform (die durch die Materialstärke von der koronal schauenden Plattformoberfläche beabstandete Oberfläche der Plattform) vorgesehen.

Die Erfindung sieht vor, dass die dreidimensionale Geometrie des Abutments unterhalb der Präparationsgrenze zumindest in dem Ausmaß durch den Bediener gewählt werden kann, dass der Bediener ausgehend vom definierten Emergenzprofil, eine Materialstärke der Plattform des Abutments (d. h. den Normalabstand zwischen der koronal schauenden Plattformoberfläche und der von dieser abgewandten Plattformoberfläche) wählen kann.

In bevorzugten Ausführungsbeispielen sieht die Erfindung vor, dass die dreidimensionale Geometrie des Abutments unterhalb der Präparationsgrenze weiters in dem Ausmaß durch den Bediener gewählt werden kann, dass durch einen Bediener des Computers
- eine Materialstärke einer Wandung des Schraubenkanals (d. h. den Normalabstand zwischen der den Schraubenkanal definierenden Innenwandung und einer von dieser abgewandten Wandung des den Schraubenkanal aufweisenden, koronal verlaufenden Bereichs des Abutments) und/oder
- eine Materialstärke der Basis des Abutments (d. h. den Normalabstand zwischen der der Anschlussstruktur zugewandten Außenseite des Abutments und der koronal schauenden Plattformoberfläche) und/oder
- für den Übergang zwischen der koronal schauenden Plattformoberfläche und der Basis des Abutments und/oder für den Übergang zwischen der Basis des Abutments und einer Wandung des Schraubenkanals ein Übergangsradius
innerhalb eines in einem elektronischen Speicher hinterlegten, vorgegebenen Bereichs gewählt wird.

Es sei angemerkt, dass konstante Materialstärken für die Materialstärke der Plattform des Abutments und/oder für die Materialstärke der Wandung des Schraubenkanals und/oder für die Materialstärke der Basis des Abutments bevorzugt werden. Grundsätzlich wäre allerdings auch ein Verlauf für eine oder mehrere dieser Materialstärken (veränderliche Materialstärke) möglich. Der Verlauf kann durch Vorgabe einer oder mehrerer Stützstellen definiert sein.

Bevorzugt ist vorgesehen, dass der jeweilige Bereich für die mögliche Wahl der Materialstärke bzw. für den Übergangsradius in Abhängigkeit eines zu verwendenden Materials des Abutments (z. B. Keramik, Titan, Kunststoff oder Hybridmaterialien) und/oder in Abhängigkeit der definierten dreidimensionalen Geometrie des Abutments oberhalb der Präparationsgrenze gewählt wird.

Der in einem elektronischen Speicher hinterlegte, vorgegebene Bereich (auf den der Computer während der Ausführung des Verfahrens, zumindest während des entsprechenden Schritts, natürlich Zugriff haben muss) kann jeweils auf Basis von Erfahrungswerten und/oder auf Basis von Simulationen und/oder Messreihen festgelegt werden. Beispielsweise kann vorgesehen sein, dass die Materialstärke
- der Basis des Abutments in einem Bereich von etwa 0,05 mm bis etwa 4 mm wählbar ist
- der Plattform in einem Bereich von etwa 0,05 mm bis etwa 2 mm wählbar ist
- der Wandung des Schraubenkanals in einem Bereich von etwa 0,05 mm bis etwa 3 mm wählbar ist

Bevorzugt ist vorgesehen, dass das Verfahren und ein entsprechendes Computerprogramm, welches bei der Ausführung des Programms durch einen Computer diesen veranlassen, das Verfahren auszuführen, ausgehend von einer bereitgestellten durch den Computer lesbaren dreidimensionalen Darstellung eines Kiefers des Patienten unmittelbar ein dreidimensionales Computermodell für ein Abutment mit tellerartig ausgebildeter koronal schauender Plattformoberfläche erstellen.

Alternativ kann aber in einer zweiten Variante der Erfindung vorgesehen sein, dass mit einem herkömmlichen Verfahren und einem entsprechenden Computerprogramm zunächst ein dreidimensionales Start-Computermodell eines patientenindividuellen Abutments erstellt wird, welches eine solche Form aufweist, dass ein sich radial zwischen dem Schraubenkanal des Abutments und dem Emergenzprofil des Abutments erstreckender Bereich als mit Material gefüllt dargestellt ist. Dann führt der Computer entsprechend dem erfindungsgemäßen Verfahren oder mit einem entsprechenden erfindungsgemäßen Computerprogramm eine Transformation durch, um aus dem dreidimensionalen Start-Computermodell das dreidimensionale Computermodell zu erhalten, bei welchem unterhalb der Präparationsgrenze für die dreidimensionale Geometrie der Plattform eine tellerartige Ausbildung der koronal schauenden Plattformoberfläche vorgesehen ist, und welches die durch einen Bediener des Computers ausgehend vom definierten Emergenzprofil gewählte Materialstärke der Plattform des Abutments aufweist. Es können dieselben zusätzlichen optionalen Merkmale vorgesehen sein, wie in Bezug auf die erste Variante der Erfindung diskutiert.

Für die Herstellung eines erfindungsgemäßen Abutments für ein Zahnimplantat unter Verwendung eines nach einem Verfahren nach wenigstens einem der vorangehenden Ausführungsbeispiele hergestellten, materialreduzierten dreidimensionalen Computermodells des patientenindividualisierten Abutments wird bevorzugt ein additives Fertigungsverfahren (z. B. Lasersintern) vorgeschlagen, bei welchem schichtweise Pulver (z. B. aus Keramik, Titan, Kunststoff oder Hybridmaterialien) durch Energieeinwirkung verfestigt wird.

Gegenüber herkömmlichen, spanabhebenden oder schneidenden Verfahren zur Herstellung eines Abutments zeichnen sich additive Fertigungsverfahren durch eine verkürzte Verfahrensdauer aus. Außerdem ist bei einem spanabhebenden oder schneidenden Verfahren häufig ein aufwändiges Umspannen erforderlich. Auch bei Einsatz eines additiven Fertigungsverfahrens kann eine spanabhebende oder schneidende Nachbehandlung erforderlich sein.

Der Schraubenkanal kann bei allen Ausführungsbeispielen parallel zu oder in einem Winkel zu einer zentralen gedachten Achse der Anschlussstruktur des Abutments verlaufen.

Ausführungsbeispiele der Erfindung werden anhand der Figuren diskutiert. Es zeigen:
- Fig. 1a,b: schematische Darstellungen zweier Varianten einer Anordnung zur Durchführung des erfindungsgemäßen Verfahrens

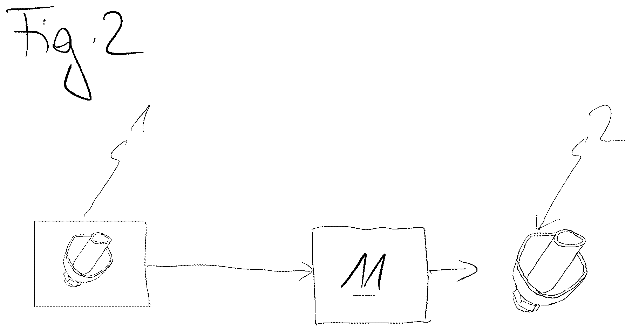

- Fig. 2: eine schematische Darstellung eines Fertigungsverfahrens eines Abutments unter Verwendung des Ergebnisses des Verfahrens der Fig. 1a oder 1b
- Fig. 3a-d: verschiedene Ansichten und eine Schnittdarstellung eines Ausführungsbeispiels eines durch die Erfindung herzustellenden Abutments
- Fig. 4: eine Schnittdarstellung wie in Fig. 3d mit Bemaßung der Materialstärken
- Fig. 5a-d: verschiedene Ansichten und eine Schnittdarstellung eines Ausführungsbeispiels eines dreidimensionales Start-Computermodells eines patientenindividuellen Abutments, welches im Zuge des erfindungsgemäßen Verfahrens transformiert wird
- Fig. 6: eine Schnittdarstellung durch ein Ausführungsbeispiel eines durch die Erfindung herzustellenden Abutments, wobei erkennbar ist, wie bei der zweiten Variante der Erfindung die Transformation ausgehend von einem dreidimensionalen Start-Computermodell erfolgt ist
- Fig. 7a-d: eine Darstellung eines durch einen Computer lesbaren dreidimensionalen Darstellung eines Kiefers eines Patienten mit einem Computermodell eines Abutments bzw. ohne das Abutment, eine Gingivamaske und ein nach dem erfindungsgemäßen Verfahren hergestelltes Abutment mit einer prothetischen Versorgung in Form einer Krone

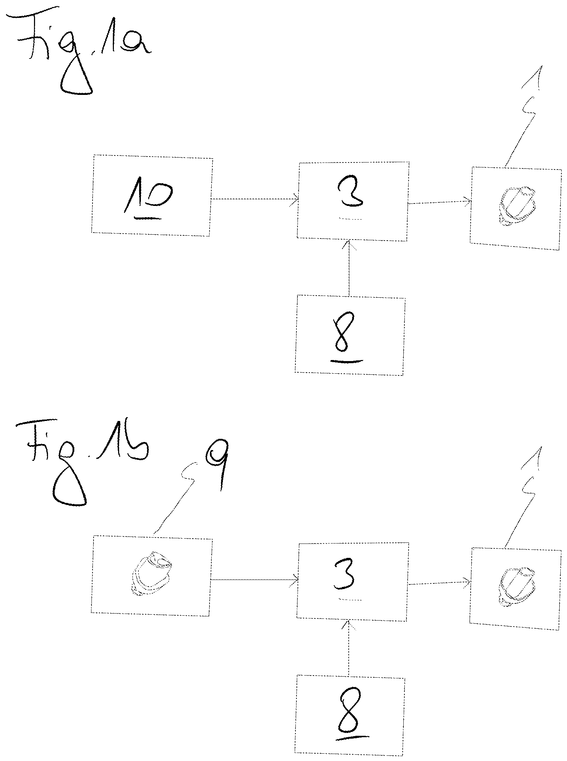

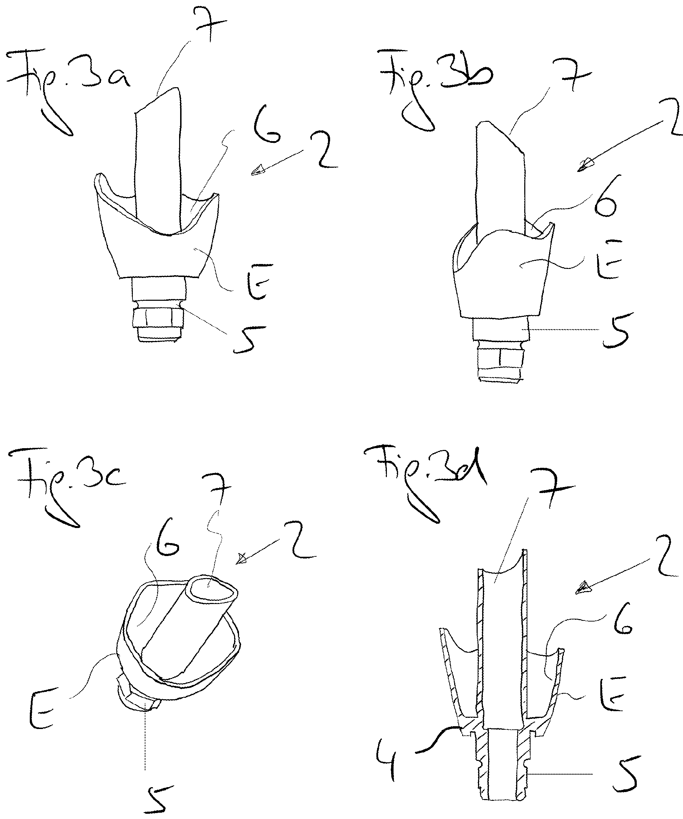

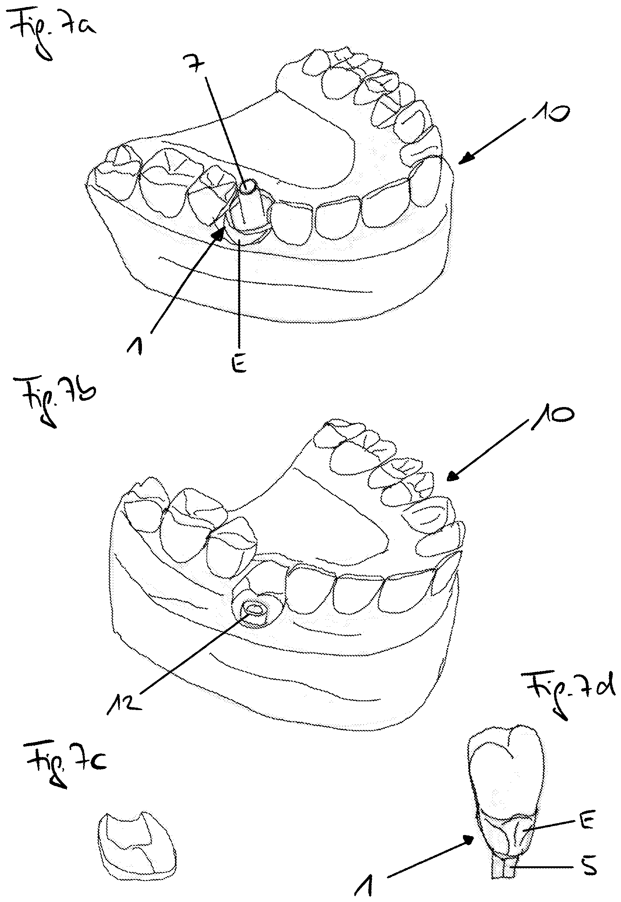

Fig. 1a zeigt schematisch eine Anordnung zum Betreiben eines Verfahrens zur Herstellung eines dreidimensionalen Computermodells 1 eines für einen Patienten bestimmten patientenindividuellen Abutments 2 für ein Zahnimplantat 12 mittels eines Computers 3, wobei das Abutment 2 (vgl. Fig. 3a-d) aufweist:
- eine Basis 4
- eine Anschlussstruktur 5 zum Anschließen des Abutments 2 an ein stiftförmiges Zahnimplantat 12 (vgl. Fig. 7b)
- eine koronal schauende Plattformoberfläche 6 einer Plattform des Abutments 2 zum Abstützen einer prothetischen Versorgung und
- einen Schraubenkanal 7 zur Befestigung des Abutments 2 an ein stiftförmiges Zahnimplantat 12

Zu erkennen ist eine zentrale gedachte Achse Z der Anschlussstruktur 5 des Abutments 2.

Es erfolgt das Bereitstellen einer durch den Computer 3 lesbaren dreidimensionalen Darstellung 10 eines Kiefers des Patienten zumindest in jenem Bereich des Kiefers, wo das Abutment 2 zu platzieren ist. Diese dreidimensionale Darstellung kann in bekannter Weise entweder auf Basis eines analogen Kieferabdrucks des Kiefers des Patienten, welcher digitalisiert wird oder unmittelbar in digitaler Form (z. B. unter Verwendung eines intraoralen Scanners und/oder eines Laborscanners) bereitgestellt werden.

In bekannter Weise erfolgt ein Wählen einer Definition einer Präparationsgrenze in Abhängigkeit eines für den Patienten individuellen Zahnfleischverlaufs dort, wo das Abutment 2 zu platzieren ist, durch einen Bediener des Computers 3 oder durch einen Algorithmus.

Es erfolgt eine Definition einer dreidimensionalen Geometrie des Abutments 2 oberhalb der Präparationsgrenze durch einen Bediener des Computers 3 oder durch einen Algorithmus. Die Definition der dreidimensionalen Geometrie des Abutments 2 kann umfassen:
- Definition einer Lage des im Abutment 2 verlaufenden Schraubenkanals 7 zur Befestigung des Abutments 2 an ein stiftförmiges Zahnimplantat 12. Üblicherweise ist die Lage relativ zu einer Ausrichtung des stiftförmigen Zahnimplantats 12 definiert.
- Definition einer Breite einer koronal schauenden Plattformoberfläche 6 des Abutments 2
- Definition einer Höhe des Abutments 2
- Definition einer Breite einer den Schraubenkanal 7 aufweisenden zentralen Erhebung des Abutments 2

Es erfolgt eine Definition des Emergenzprofils E des Abutments 2 durch einen Bediener des Computers 3 oder durch einen Algorithmus.

Erfindungsgemäß ist unterhalb der Präparationsgrenze für die dreidimensionale Geometrie der Plattform eine tellerartige Ausbildung der koronal schauenden Plattformoberfläche 6 vorgesehen.

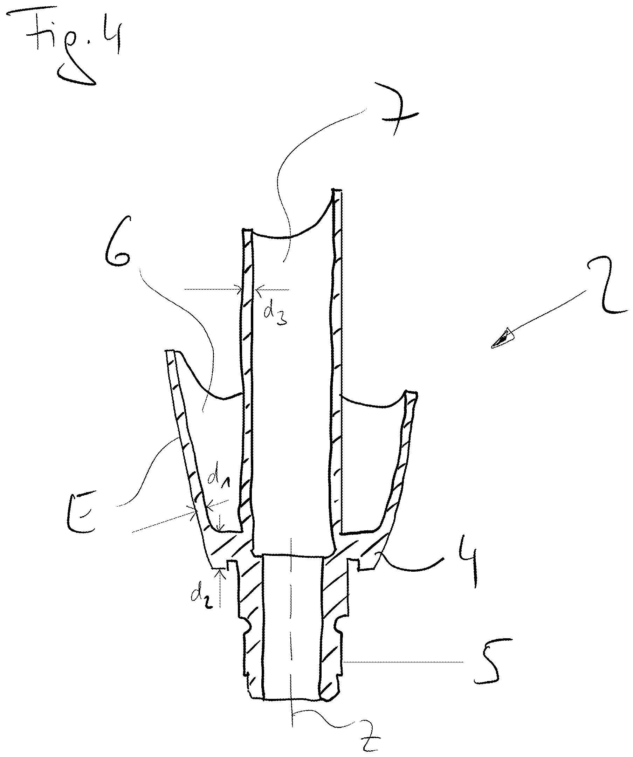

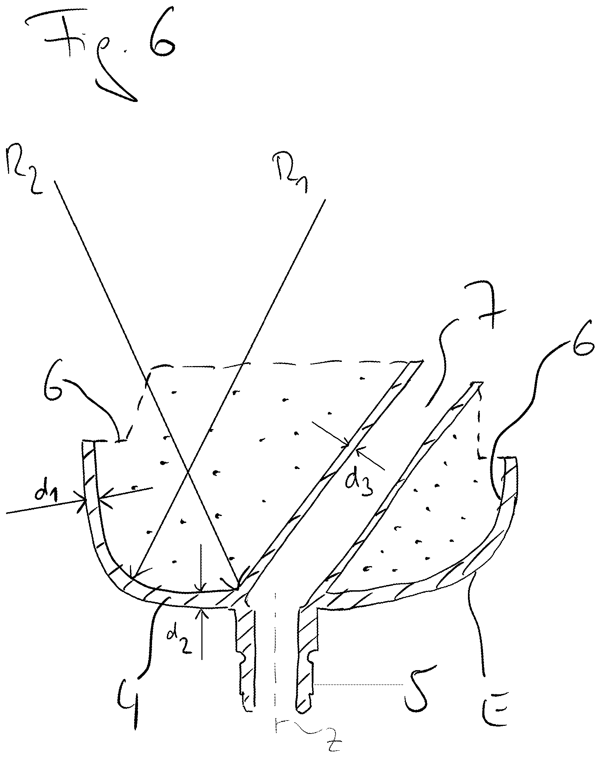

Durch einen Bediener des Computers 3 werden ausgehend vom definierten Emergenzprofil E innerhalb von in einem elektronischen Speicher 8 hinterlegten vorgegebenen Bereichen gewählt (vgl. Fig. 4 und 6):
- eine Materialstärke d₁ der Plattform des Abutments 2
- eine Materialstärke d₂ der Basis 4 des Abutments 2 und/oder
- eine Materialstärke d₃ einer Wandung des Schraubenkanals 7 und/oder
- einen Übergangsradius R₁ für den Übergang zwischen der koronal schauenden Plattformoberfläche 6 und der Basis 4 des Abutments 2
- einen Übergangsradius R₂ für den Übergang zwischen der Basis 4 des Abutments 2 und einer Wandung des Schraubenkanals 7

Das dreidimensionale Computermodell 1 wird durch den Computer 3 bereitgestellt, beispielsweise zur Herstellung eines patientenindividuellen Abutments 2 für ein Zahnimplantat 12 unter Verwendung eines nach dem soeben beschriebenen Verfahren hergestellten dreidimensionalen Computermodells 1, vorzugsweise durch ein additives Fertigungsverfahren.

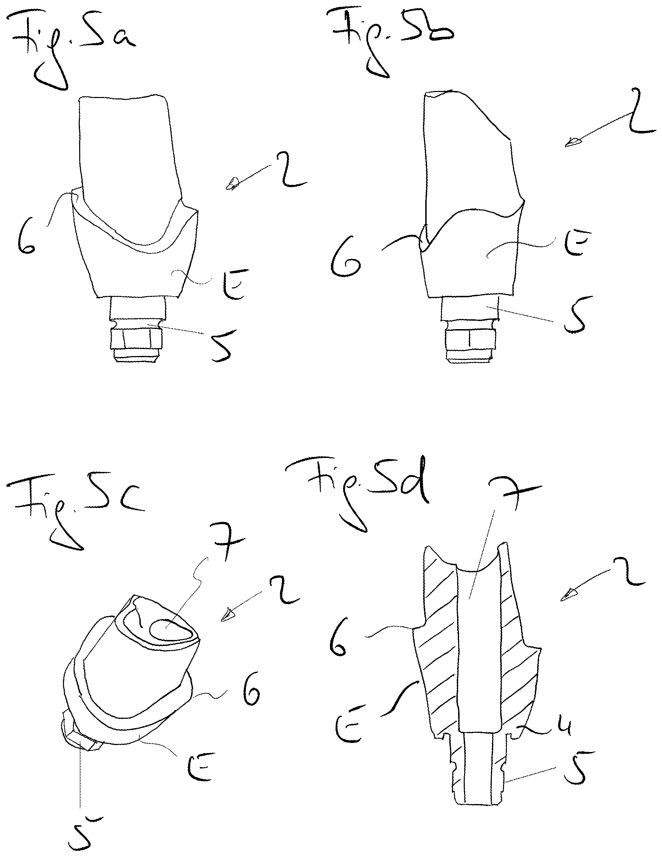

Das Ausführungsbeispiel der Fig. 1b unterscheidet sich von dem bisher diskutierten Ausführungsbeispiel nur dadurch, dass entsprechend der zweiten Variante der Erfindung zunächst in Abhängigkeit der bereitgestellten dreidimensionalen Darstellung des Kiefers, der Definition des Emergenzprofils E und der dreidimensionalen Geometrie des Abutments 2 oberhalb der Präparationsgrenze ein dreidimensionales Start-Computermodell 9 eines patientenindividuellen Abutments 2 erstellt wird, wobei das dreidimensionale Start-Computermodell 9 eine solche Form aufweist, dass ein sich radial zwischen dem Schraubenkanal 10 des Abutments 2 und dem Emergenzprofil E des Abutments 2 erstreckender Bereich als mit Material gefüllt dargestellt ist (vgl. Fig. 5a-d). Ausgehend von diesem dreidimensionalen Start-Computermodell 9 (welches an sich ein funktionsfähiges Abutment repräsentiert) führt der Computer 3 eine durch einen Algorithmus (der Algorithmus errechnet z.B. je nach Winkel des Schraubenkanals eine gewisse notwendige Materialstärke je nach errechneter Belastungsspitzen) Transformation durch, um aus dem dreidimensionalen Start-Computermodell 9 das dreidimensionale Computermodell 1 zu erhalten, bei welchem unterhalb der Präparationsgrenze für die dreidimensionale Geometrie der Plattform eine tellerartige Ausbildung der koronal schauenden Plattformoberfläche 6 vorgesehen ist und welches die durch einen Bediener des Computers 3 ausgehend vom definierten Emergenzprofil E gewählte Materialstärke d₁ der Plattform des Abutments 2 und die weiteren diskutierten Parameter aufweist. In Fig. 6 ist beispielhaft dargestellt, welcher als mit Material gefüllt dargestellter Bereich (gepunkteter Bereich) bei der Transformation zu entfernen ist.

Fig. 7a zeigt eine Darstellung eines durch den Computer 3 lesbaren dreidimensionalen Darstellung 10 eines Kiefers eines Patienten mit einem dreidimensionalen Computermodell 1 eines patientenindividuellen Abutments 2.

In Fig. 7b wurde das in Fig. 7a gezeigte Abutment 2 entfernt, was eine Ansicht des oberen Bereichs eines stiftförmigen Zahnimplantats 12 gestattet. In diesen oberen Bereich des stiftförmigen Zahnimplantats 12 wird das Abutment 2 mit seiner Anschlussstruktur 5 eingesetzt. Fig. 7c zeigt eine Gingivamaske (welche in Fig. 7a und 7b allerdings nicht vorgesehen ist).

Fig. 7d zeigt ein nach dem erfindungsgemäßen Verfahren hergestelltes Abutment 2 mit einer prothetischen Versorgung in Form einer Krone.

### Bezugszeichenliste:

- 1: dreidimensionales Computermodell eines patientenindividuellen Abutments
- 2: Abutment
- 3: Computer
- 4: Basis des Abutments
- 5: Anschlussstruktur
- 6: koronal schauende Plattformoberfläche
- 7: Schraubenkanal
- 8: elektronischer Speicher
- 9: dreidimensionales Start-Computermodell eines patientenindividuellen Abutments
- 10: dreidimensionale Darstellung eines Kiefers des Patienten
- 11: Fertigungsanlage
- 12: Zahnimplantat

- E: Emergenzprofil des Abutments
- Z: zentrale gedachte Achse der Anschlussstruktur des Abutments
- d₁: Materialstärke der Plattform des Abutments
- d₂: Materialstärke der Basis des Abutments
- d₃: Materialstärke einer Wandung des Schraubenkanals des Abutments
- R₁: Übergangsradius eines Übergangs zwischen der koronal schauenden Plattformoberfläche und der Basis des Abutments
- R₂: Übergangsradius eines Übergangs zwischen der Basis des Abutments und einer Wandung des Schraubenkanals

## Patentansprüche

1. Verfahren zur Herstellung eines dreidimensionalen Computermodells (1) eines für einen Patienten bestimmten patientenindividuellen Abutments (2) für ein Zahnimplantat (12) mittels eines Computers (3), wobei das Abutment (2) zumindest eine Basis (4), eine Anschlussstruktur (5) zum Anschließen des Abutments (2) an ein stiftförmiges Zahnimplantat (12), eine koronal schauende Plattformoberfläche (6) einer Plattform des Abutments (2) zum Abstützen einer prothetischen Versorgung und einen Schraubenkanal (7) zur Befestigung des Abutments (2) an ein stiftförmiges Zahnimplantat (12) aufweist, umfassend wenigstens folgende Schritte:
- Bereitstellen einer durch den Computer (3) lesbaren dreidimensionalen Darstellung (10) eines Kiefers des Patienten zumindest in jenem Bereich des Kiefers, wo das Abutment (2) zu platzieren ist
- Wählen einer Definition einer Präparationsgrenze in Abhängigkeit eines für den Patienten individuellen Zahnfleischverlaufs dort, wo das Abutment (2) zu platzieren ist, durch einen Bediener des Computers (3) oder durch einen Algorithmus
- Definition einer dreidimensionalen Geometrie des Abutments (2) oberhalb der Präparationsgrenze durch einen Bediener des Computers (3) oder durch einen Algorithmus
- Definition eines Emergenzprofils (E) des Abutments (2) durch einen Bediener des Computers (3) oder durch einen Algorithmus
- Bereitstellen des dreidimensionalen Computermodells (1) durch den Computer (3)
**dadurch gekennzeichnet, dass** unterhalb der Präparationsgrenze für die dreidimensionale Geometrie der Plattform eine tellerartige Ausbildung der koronal schauenden Plattformoberfläche (6) vorgesehen ist und durch einen Bediener des Computers (3) ausgehend vom definierten Emergenzprofil (E) eine Materialstärke (d₁) der Plattform des Abutments (2) innerhalb eines in einem elektronischen Speicher (8) hinterlegten, vorgegebenen Bereichs gewählt wird.

2. Verfahren zur Herstellung eines dreidimensionalen Computermodells (1) eines für einen Patienten bestimmten patientenindividuellen Abutments (2) für ein Zahnimplantat (12) mittels eines Computers (3), wobei das dreidimensionale Start-Computermodell (9) ein Abutment repräsentiert, welches eine Basis (4), eine Anschlussstruktur (5) zum Anschließen des Abutments (2) an ein stiftförmiges Zahnimplantat (12), eine koronal schauende Plattformoberfläche (6) einer Plattform des Abutments (2) zum Abstützen einer prothetischen Versorgung und einen Schraubenkanal (7) zur Befestigung des Abutments (2) an ein stiftförmiges Zahnimplantat (12) aufweist, **gekennzeichnet durch** wenigstens folgende Schritte:
- Bereitstellen eines dreidimensionalen Start-Computermodells (9) des patientenindividuellen Abutments (2), wobei das dreidimensionale Start-Computermodell (9) eine solche Form aufweist, dass ein sich radial zwischen dem Schraubenkanal (10) des Abutments (2) und dem Emergenzprofil (E) des Abutments (2) erstreckender Bereich als mit Material gefüllt dargestellt ist
- Durchführen einer Transformation mittels des Computers (3), um aus dem dreidimensionalen Start-Computermodell (9) ein dreidimensionales Computermodell (1) zu erhalten, bei welchem unterhalb der Präparationsgrenze für die dreidimensionale Geometrie der Plattform eine tellerartige Ausbildung der koronal schauenden Plattformoberfläche (6) vorgesehen ist, und welches eine durch einen Bediener des Computers (3) ausgehend vom definierten Emergenzprofil (E) innerhalb eines in einem elektronischen Speicher (8) hinterlegten, vorgegebenen Bereichs gewählte Materialstärke (d₁) der Plattform des Abutments (2) aufweist.

3. Verfahren nach einem der beiden vorangehenden Ansprüche, wobei durch einen Bediener des Computers (3) weiters eine Materialstärke (d₃) einer Wandung des Schraubenkanals (7) innerhalb eines in einem elektronischen Speicher (8) hinterlegten, vorgegebenen Bereichs gewählt wird.

4. Verfahren nach wenigstens einem der vorangehenden Ansprüche, wobei durch einen Bediener des Computers (3) eine Materialstärke (d₂) der Basis (4) des Abutments (2) innerhalb eines in einem elektronischen Speicher (8) hinterlegten, vorgegebenen Bereichs gewählt wird.

5. Verfahren nach wenigstens einem der vorangehenden Ansprüche, wobei
- für den Übergang zwischen der koronal schauenden Plattformoberfläche (6) und der Basis (4) des Abutments (2) und/oder
- für den Übergang zwischen der Basis (4) des Abutments (2) und einer Wandung des Schraubenkanals (7)
ein Übergangsradius (R₁, R₂) innerhalb eines in einem elektronischen Speicher (8) hinterlegten, vorgegebenen Bereichs gewählt wird.

6. Verfahren nach wenigstens einem der vorangehenden Ansprüche, wobei der jeweilige Bereich für die mögliche Wahl der Materialstärke (d₁, d₂, d₃) bzw. für den Übergangsradius (R₁, R₂) in Abhängigkeit eines zu verwendenden Materials des Abutments (2) und/oder in Abhängigkeit der definierten dreidimensionalen Geometrie des Abutments (2) oberhalb der Präparationsgrenze gewählt wird.

7. Verfahren zur Herstellung eines patientenindividuellen Abutments (2) für ein Zahnimplantat (12) unter Verwendung eines nach einem Verfahren nach wenigstens einem der vorangehenden Ansprüche hergestellten dreidimensionalen Computermodells (1), vorzugsweise durch ein additives Fertigungsverfahren.

8. Computerprogrammprodukt, umfassend Befehle, die bei der Ausführung des Programms durch einen Computer (4) diesen veranlassen, das Verfahren nach wenigstens einem der Ansprüche 1 bis 6 auszuführen.

9. Computerlesbares Speichermedium, umfassend Befehle, die bei der Ausführung durch einen Computer (4) diesen veranlassen, das Verfahren nach wenigstens einem der Ansprüche 1 bis 6 auszuführen.

10. Computerlesbarer Datenträger, auf dem das Computerprogrammprodukt nach Anspruch 8 gespeichert ist.

11. Datenträgersignal, das das Computerprogrammprodukt nach Anspruch 8 überträgt.

12. Computer, welcher zur Durchführung eines Verfahrens nach wenigstens einem der Ansprüche 1 bis 7 und/oder zur Ausführung eines Computerprogrammprodukts nach Anspruch 8 konfiguriert ist.

## Claims

1. A method of producing a three-dimensional computer model (1) of a patient-individual abutment (2) for a dental implant (12) specific for a patient by means of a computer (3), wherein the abutment (2) has at least a base (4), a connecting structure (5) for connecting the abutment (2) to a pin-shaped dental implant (12), a coronally looking platform surface (6) of a platform of the abutment (2) for supporting a prosthetic fitting and a screw passage (7) for fixing the abutment (2) to a pin-shaped dental implant (12), comprising at least the following steps:
- providing a three-dimensional representation (10) of a jaw of the patient readable by the computer (3), at least in that region of the jaw where the abutment (2) is to be placed,
- selecting a definition of a preparation limit in dependence on a gum line individual for the patient where the abutment (2) is to be placed, by an operator of the computer (3) or by an algorithm,
- defining a three-dimensional geometry of the abutment (2) above the preparation limit by an operator of the computer (3) or by an algorithm,
- defining an emergence profile (E) of the abutment (2) by an operator of the computer (3) or by an algorithm, and
- providing the three-dimensional computer model (1) by the computer (3),
**characterised in that** provided below the preparation limit for the three-dimensional geometry of the platform is a plate-like configuration of the coronally looking platform surface (6) and a material thickness (d₁) of the platform of the abutment (2) is selected by an operator of the computer (3) starting from the defined emergence profile (E) within a predetermined range stored in an electronic memory (8).

2. A method of producing a three-dimensional computer model (1) of a patient-individual abutment (2) for a dental implant (12) specific for a patient by means of a computer (3), wherein the three-dimensional start computer model (9) represents an abutment which has a base (4), a connecting structure (5) for connecting the abutment (2) to a pin-shaped dental implant (12), a coronally looking platform surface (6) of a platform of the abutment (2) for supporting a prosthetic fitting and a screw passage (7) for fixing the abutment (2) to a pin-shaped dental implant (12), **characterised by** at least the following steps:
- providing a three-dimensional start computer model (9) of the patient-individual abutment (2), wherein the three-dimensional start computer model (9) is of such a form that a region extending radially between the screw passage (10) of the abutment (2) and the emergence profile (E) of the abutment (2) is represented as filled with material,
- carrying out a transformation by means of the computer (3) in order to obtain from the three-dimensional start computer model (9) a three-dimensional computer model (1) in which a plate-like configuration of the coronally looking platform surface (6) is provided below the preparation limit for the three-dimensional geometry of the platform and which has a material thickness (d₁) of the platform of the abutment (2) that is selected by an operator of the computer (3) starting from the defined emergence profile (E) within a predetermined range stored in an electronic memory (8).

3. A method as set forth in one of the two preceding claims wherein further a material thickness (d₃) of a wall of the screw passage (7) is selected by an operator of the computer (3)within a predetermined region stored in an electronic memory (8).

4. A method as set forth in one of the preceding claims wherein a material thickness (d₂) of the base (4) of the abutment (2) is selected by an operator of the computer (3) within a predetermined range stored in an electronic memory (8).

5. A method as set forth in at least one of the preceding claims wherein a transition radius (R₁, R₂) within a predetermined region stored in an electronic memory (8) is selected
- for the transition between the coronally looking platform surface (6) and the base (4) of the abutment (2), and/or
- for the transition between the base (4) of the abutment (2) and a wall of the screw passage (7).

6. A method as set forth in at least one of the preceding claims wherein the respective range for the possible selection of the material thickness (d₁, d₂, d₃) or for the transition radius (R₁, R₂), respectively, is selected in dependence on a material to be used for the abutment (2) and/or in dependence on the defined three-dimensional geometry of the abutment (2) above the preparation limit.

7. A method of producing a patient-individual abutment (2) for a dental implant (12) using a three-dimensional computer model (1) produced in accordance with a method as set forth in one of the preceding claims, preferably by an additive production method.

8. A computer program product comprising commands which when the program is executed by a computer (4) cause it to carry out the method as set forth in at least one of claims 1 through 6.

9. A computer-readable storage medium comprising commands which when executed by a computer (4) cause it to carry out the method as set forth in at least one of claims 1 through 6.

10. A computer-readable data carrier on which the computer program product as set forth in claim 8 is stored.

11. A data carrier signal which transmits the computer program product as set forth in claim 8.

12. A computer configured to carry out a method as set forth in at least one of claims 1 through 7 and/or for executing a computer program product as set forth in claim 8.

## Revendications

1. Procédé de fabrication d'un modèle informatique tridimensionnel (1) d'une butée (2) personnalisée destinée à un patient pour un implant dentaire (12) au moyen d'un ordinateur (3), dans lequel la butée (2) présente au moins une base (4), une structure de raccordement (5) pour raccorder la butée (2) à un implant dentaire (12) en forme de broche, une surface de plateforme (6) orientée coronalement d'une plateforme de la butée (2) pour soutenir un traitement prothétique et un canal de vis (7) pour fixer la butée (2) à un implant dentaire (12) en forme de broche, comprenant au moins des étapes suivantes :
- de fourniture d'une représentation tridimensionnelle (10) lisible par l'ordinateur (3) d'une mâchoire du patient au moins dans la zone de la mâchoire où la butée (2) doit être placée,
- de sélection d'une définition d'une marge de préparation en fonction d'un tracé des gencives personnalisé pour le patient à l'endroit où la butée (2) doit être placée, par un opérateur de l'ordinateur (3) ou par un algorithme,
- de définition d'une géométrie tridimensionnelle de la butée (2) au-dessus de la marge de préparation par un opérateur de l'ordinateur (3) ou par un algorithme,
- de définition d'un profil d'émergence (E) de la butée (2) par un opérateur de l'ordinateur (3) ou par un algorithme,
- de fourniture du modèle informatique tridimensionnel (1) par l'ordinateur (3),
**caractérisé en ce que**, en dessous de la marge de préparation pour la géométrie tridimensionnelle de la plateforme, une réalisation en forme de disque de la surface de plateforme (6) orientée coronalement est prévue et une épaisseur de matériau (d₁) de la plateforme de la butée (2) est sélectionnée par un opérateur de l'ordinateur (3) à partir du profil d'émergence (E) défini dans une plage prédéfinie enregistrée dans une mémoire électronique (8).

2. Procédé de fabrication d'un modèle informatique tridimensionnel (1) d'une butée (2) personnalisée destinée à un patient pour un implant dentaire (12) au moyen d'un ordinateur (3), dans lequel le modèle informatique de départ tridimensionnel (9) représente une butée qui présente une base (4), une structure de raccordement (5) pour raccordée la butée (2) à un implant dentaire (12) en forme de broche, une surface de plateforme (6) orientée coronalement d'une plateforme de la butée (2) pour soutenir un traitement prothétique et un canal de vis (7) pour fixer la butée (2) à un implant dentaire (12) en forme de broche, **caractérisé par** au moins des étapes suivantes :
- de fourniture d'un modèle informatique de départ tridimensionnel (9) de la butée (2) personnalisée, dans lequel le modèle informatique de départ tridimensionnel (9) présentant une forme telle qu'une zone s'étendant radialement entre le canal de vis (10) de la butée (2) et le profil d'émergence (E) de la butée (2) est représentée comme étant remplie de matériau,
- de réalisation d'une transformation au moyen de l'ordinateur (3) pour obtenir à partir du modèle informatique de départ tridimensionnel (9) un modèle informatique tridimensionnel (1) dans lequel, en dessous de la marge de préparation pour la géométrie tridimensionnelle de la plateforme, une réalisation en forme de disque de la surface de plateforme (6) orientée coronalement est prévue, et qui présente une épaisseur de matériau (d₁) sélectionnée de la plateforme de la butée (2) sélectionnée par un opérateur de l'ordinateur (3) à partir du profil d'émergence (E) défini à l'intérieur d'une plage prédéfinie enregistrée dans une mémoire électronique (8).

3. Procédé selon l'une quelconque des deux revendications précédentes, dans lequel une épaisseur de matériau (d₃) d'une paroi du canal de vis (7) est en outre sélectionnée dans une plage prédéfinie enregistrée dans une mémoire électronique (8) par un opérateur de l'ordinateur (3).

4. Procédé selon au moins l'une des revendications précédentes, dans lequel une épaisseur de matériau (d₂) de la base (4) de la butée (2) est sélectionnée dans une plage prédéfinie enregistrée dans une mémoire électronique (8) par un opérateur de l'ordinateur (3).

5. Procédé selon au moins l'une quelconque des revendications précédentes, dans lequel
- pour la transition entre la surface de plateforme (6) orientée coronalement et la base (4) de la butée (2) et/ou
- pour la transition entre la base (4) de la butée (2) et une paroi du canal à vis (7)
un rayon de transition (R₁, R₂) est sélectionné dans une plage prédéfinie enregistrée dans une mémoire électronique (8).

6. Procédé selon au moins l'une quelconque des revendications précédentes, dans lequel la plage respective pour la sélection possible de l'épaisseur de matériau (d₁, d₂, d₃) ou pour le rayon de transition (R₁, R₂) est sélectionnée en fonction d'un matériau à utiliser de la butée (2) et/ou en fonction de la géométrie tridimensionnelle définie de la butée (2) au-dessus de la marge de préparation.

7. Procédé de fabrication d'une butée (2) personnalisée pour un implant dentaire (12) à l'aide d'un modèle informatique tridimensionnel (1) fabriqué selon un procédé selon au moins l'une des revendications précédentes, de préférence par un procédé de fabrication additive.

8. Produit-programme d'ordinateur comprenant des instructions qui, lors de l'exécution du programme par un ordinateur (4), amènent celui-ci à exécuter le procédé selon au moins l'une quelconque des revendications 1 à 6.

9. Support de stockage lisible par ordinateur, comprenant des instructions qui, lorsqu'elles sont exécutées par un ordinateur (4), amènent celui-ci à exécuter le procédé selon au moins l'une quelconque des revendications 1 à 6.

10. Support de données lisible par ordinateur sur lequel le produit-programme d'ordinateur selon la revendication 8 est stocké.

11. Signal de support de données qui est transmis par le produit-programme d'ordinateur selon la revendication 8.

12. Ordinateur qui est configuré pour réaliser un procédé selon au moins l'une quelconque des revendications 1 à 7 et/ou pour exécuter un produit-programme d'ordinateur selon la revendication 8.
